# EUROPEAN PATENT SPECIFICATION

(11) **EP 4 237 822 B1**
(45) Date of publication and mention of the grant of the patent: **02.07.2025**
(21) Application number: 22731712.0
(22) Date of filing: 08.06.2022
(51) Int. Cl.: G01N 15/02, C04B 28/04, C04B 40/00, G01N 33/24

(54) **DIGITAL PARTICLE ANALYSIS**
DIGITALE PARTIKELANALYSE
ANALYSE NUMÉRIQUE DES PARTICULES

(30) Priority: 08.06.2021 WO PCT/EP2021/065367; 24.01.2022 EP 22153044
(43) Date of publication of application: 06.09.2023
(60) Divisional application: 25171971.2
(73) Proprietor: Sika Technology AG, 6340 Baar (CH)
(72) Inventor: VELTEN, Ulf, 8955 Oetwil an der Limmat (CH); VOGELSANG, Jörg, 5505 Brunegg (CH); WEINKAUF, Annette, 8048 Zürich (CH); ZIMMERMANN, Jörg, 8400 Winterthur (CH); RIEGER, Carsten, 8046 Zürich (CH); LINDLAR, Benedikt, 78467 Konstanz (DE); APEH, Christopher, 8910 Affoltern am Albis (CH); MARAZZINI, Oscar, 20034 San Giorgio su Legnano (IT); OBST, Stefan, 8055 Zürich (CH); VORWERK, Michael, 8115 Hüttikon (CH); LISKA, Martin, Cambridge Cambridgeshire CB23 8ST (GB); KUNDRA, Milan, Hertfordshire EN5 5QY (GB)
(74) Representative: Sika Patent Attorneys
(86) International application number: PCT/EP2022/065601
(87) International publication number: WO 2022/258715

(56) References cited:
- GB-A- 2 524 130
- US-A1- 2018 068 825
- US-A1- 2020 340 804
- ARAUJO GEORGIA S. ET AL: "Use of digital image analysis combined with fractal theory to determine particle morphology and surface texture of quartz sands", JOURNAL OF ROCK MECHANICS AND GEOTECHNICAL ENGINEERING, vol. 9, no. 6, 1 December 2017 (2017-12-01), pages 1131 - 1139, XP093122414, ISSN: 1674-7755, DOI: 10.1016/j.jrmge.2017.06.004
- SEKHAR TIWARY CHANDRA ET AL: "Onset of sphalerite to wurtzite transformation in ZnS nanoparticles", JOURNAL OF APPLIED PHYSICS, AMERICAN INSTITUTE OF PHYSICS, 2 HUNTINGTON QUADRANGLE, MELVILLE, NY 11747, vol. 110, no. 3, 1 August 2011 (2011-08-01), pages 34908 - 34908, XP012154165, ISSN: 0021-8979, [retrieved on 20110815], DOI: 10.1063/1.3622625
- NICHOLAS RUDAWSKI: "FEI Tecnai F20 S/TEM: basic operation in TEM mode", 6 September 2018 (2018-09-06), XP093216417, Retrieved from the Internet <URL:https://www.youtube.com/watch?v=noE_F1o1XmA>

## Description

### Technical field

The invention relates to a computer-implemented method for characterization of solid particles, especially sand particles

### Background art

In construction industry, curable compositions are widely used for various applications. Examples of such compositions are mortar, concrete, grout, or screed compositions. These compositions typically comprise binders in combination with solid aggregates.

Binders might be selected from mineral binders, such as e.g. hydraulic binders, as well as organic binders, e.g. curable polymer compositions. Aggregates are chosen depending on the desired properties of the curable compositions. Typically aggregates include sand, gravel, rock flour, and/or polymeric particles.

Regarding sands, for example, usually high-quality river sands have been used up to now. In the future, however, high-quality sands will become rare and hard to obtain. Thus, more and more low-quality sands have to be used, either from natural sources, e.g. low-grade river sands, or from special manufacturing processes, e.g. crushed rocks. This will have a huge impact on the formulations of curable compositions, especially concrete and mortar compositions, since the specific nature of the sands have a significant impact on the properties of the curable compositions. This has to be taken into account properly because, despite the decreasing sand quality, the technical requirements for cured compositions, e.g. hardened concrete or mortar, will remain unchanged or even be raised.

In general, in order to produce curable compositions with desired properties in a targeted manner, it is important to take into account the specific nature of the aggregates. Regarding low-quality sands, for example, it is in particular important to check if with the low-quality sand (i) the nature of fines is critical, (ii) the particle shape is critical and/or if (iii) the fines content is sufficient.

Specifically, the behavior of sand in curable compositions, especially concrete and mortar compositions, depends inter alia on the crushing technique, particle shape, surface texture, fines content, mineral contamination, and grading curve.

Grading curves of sand and aggregates is a key element to calculate the grading of a concrete mix design to ensure a dense packing of sand and aggregates. Thereby, in particular, the particle shape, which in turn depends on the production method or the sand and/or aggregate source, typically influences the needed grading of the concrete mix design.

For manufactured sand, especially items (ii) and (iii) are relevant. The fines content can be determined via sieve analysis. Although it is a standard procedure, it is very time consuming. Sieving shows if the grading curve is as desired or if it has to be modified by mixing with other sand fractions.

In contrast, the particle shape, i.e. the above-mentioned item (ii), is hardly analyzed in daily work in order to optimize the grading curve of a curable composition. This is normally only done if it is needed to control whether the aggregates comply with certain standards. This is due to the fact that analyzing the particle shape of a specific sand is very time consuming and needs special equipment, for example as defined in standard EN 933-1:2012 to -7:2012.

US 2020/340804 discloses a system for measuring an angle of repose including a device that holds granular material, and a portable smart device having a camera and a processor. The device is configured to allow part of the granular material to move freely, due to gravity, from an upper chamber to a lower chamber.

GB 2524130 discloses an analysing apparatus attachable to a smartphone or tablet device and which can interact with an image capture device of said smartphone or tablet to analyse sample material.

The analysis of particle morphology and surface texture of quartz sands by digital image analysis combined with fractal theory is described in the Article "Use of digital image analysis combined with fractal theory to determine particle morphology and surface texture of quartz sands" by G. Araujo et al in Journal of Rock Mechanics and Geotechnical Engineering, 9, 2017, 1131 - 1139.

The imaging of powder materials by transmission electron microscope is described, for example, in the Article "Onset of spheralite to wurtzite transformation in ZnS nanoparticles" by C. Tiwary et al in Journal of Applied Physics 110, 034908 (2011).

Thus, there is still a need to provide improved solutions which allow for comprehensively characterizing aggregates.

### Disclosure of the invention

It is an object of the present invention to provide improved solutions for characterizing aggregates. Aggregates include in particular sands. Thereby, preferably, the solutions should allow for a fast and reliable characterization process with a minimum of equipment required. Especially preferred, the solutions should allow for performing a complete characterization of aggregates in daily practice, in particular with regard to grading curve and their particle shape parameters. Preferably such that with the characterized aggregates, formulations of curable compositions with desired properties can be produced in a targeted manner.

Surprisingly, it has been found that the features of claim 1 achieve this object.

The inventive method is a unique approach which can be implemented on conventional mobile devices, such as e.g. smartphones. Thus, there is no need for complex and expensive analysis equipment. The method furthermore allows for a very fast, flexible and accurate digital characterization of solid particles, both in terms of size and shape. Thereby, solid particles of different nature and size can easily be characterized with one and the same hardware devices.

Thanks to the fast and easy way to carry out characterization of solid particles, daily mix design optimization for curable compositions, e.g. for concrete compositions, is possible with neglectable additional effort.

Specifically, the method can be implemented in various ways, e.g. in the form of a standalone application running on the mobile device without need for any further resources such as for example server systems. This is in particular helpful in areas with limited access to communication networks, e.g. far away from urban centers or underground. However, the method can be implemented as well in a distributed computing environment, e.g. comprising the mobile device as a client in combination with a dedicated server as a storage unit and a specialized processing unit.

Also, the inventive method can be implemented in a flexible manner with known software architectures, e.g. as native application, a progressive web application (PWA), or a hybrid application (combination of native and PWA). Thereby, helpful internet links to tutorials or support sites as well as sharing functions (e.g. via e-mail, Bluetooth, Airdrop, or others communication means) can be included in such applications as well. Also, the inventive method can be implemented in one single application or it may be divided into two or more separate applications with appropriate software interfaces for data exchange between the applications. Furthermore, the application(s) can be extended with additional functions in a flexible manner.

Applications can be implemented for any kind of operating systems such as e.g. iOS, Android, Microsoft Windows and/or Linux.

The applications can be made available easily for anyone via different distribution channels, such as e.g. public download centers (for example Apple^{®} Store and Google Play^{®}) private company operated websites and/or dedicated download sites.

Additional aspects of the invention are subject of further independent claims. Particularly preferred embodiments are outlined throughout the description and the dependent claims.

### Ways of carrying out the invention

A first aspect of the present invention is directed to a computer-implemented method for characterization of solid particles, especially sand particles, comprising the steps of:
a) Providing a sample of solid particles to be analyzed in a predefined sample area;
b) Taking at least one digital image of the sample of solid particles with a camera of a mobile computer device;
c) Performing an imaging particle analysis of the at least one digital image for extracting at least one particle size parameter, and optionally at least one particle shape parameter, preferably at least one particle size parameter and at least one particle shape parameter, of the population of particles in the at least one digital image;
d) Making available the at least one particle size parameter, and optionally the at least one particle shape parameter, preferably at least one particle size parameter and at least one particle shape parameter, via a user interface, via a machine interface and/or on a data storage medium,
wherein in step a) the sample area is a two-dimensional sample area which is aligned horizontally and wherein the solid particles are arranged in a single layer and/or such that there is essentially no overlap of particles in the sample area.

In the present context, a mobile computer device in particular is meant to be a handheld computer, i.e. a computer small enough to hold and operate in the hand of a human.

Especially the mobile computer device comprises a human interface device, especially comprising an input device and a display, and, preferably a communication interface, most preferably a wireless communication interface.

The mobile computer device in particular is selected from a mobile phone, a mobile computer or a portable computer. Especially, the mobile computer device is selected from a smartphone, a phablet, a tablet computer, a portable computer, a smartwatch, and/or a head-mounted display with camera. Highly preferred are mobile phones and/or smartphones.

Mobile phones and smartphones typically are equipped with high resolution cameras, an input device and a display. Thereby the input device and the display typically are combined in a touch sensitive display. Therefore, mobile phones and smartphones provide all of the hardware components required for performing the inventive method. Furthermore, such kind of devices can be held stably in the hand, which makes them highly suitable for taking images. At the same time mobile phones and smartphones typically have displays that are large enough for displaying complex data in well readable manner.

The expression "a camera of a mobile computer device" is meant to be an internal camera, which is integrated in the mobile computer device. In contrast, "a camera connected to a mobile computer device" means an external camera, which is a separate device connected to the mobile computer device. For example, the external camera is a camera attachable to the mobile computer device or a standalone camera. The connection between the external camera and the mobile computer device can be a wired and/or a wireless connection. A camera connected to a mobile computer device is not part of the present invention.

Preferably, the camera is a camera for taking images in the visible spectrum, especially color images. Color images allow for considering color properties of the solid particles and/or the predefined sample area in step c) of the inventive method. Thus, preferably, the at least one digital image taken in step b) is a color image. However, if color information is not required, other cameras, e.g. monochromatic cameras, can be used as well. In this case, the image is a monochromatic image, e.g. a black-and-white image.

Also, it is possible to use a camera for taking images outside the visible spectrum, e.g. in the infrared and/or ultraviolet range of the electromagnetic spectrum. Such cameras can be used alternatively or in addition to other cameras. In this case the properties of the solid particles in spectral ranges outside the visible spectrum can be considered in step c).

Preferably, the camera has a resolution of at least 2 megapixels, especially at least 5 megapixels, preferably at least 8 megapixels, particularly at least 12 megapixels, highly preferred at least 20 megapixels, even more preferred at least 50 megapixels or at least more than 100 megapixels. Especially, the camera has a resolution of at least 3'800 pixels × at least 2'100 pixels or a so called 4K, preferably 8K, more preferably 12K, especially 16K resolution. The higher the resolution the smaller solid particles can be identified in the sample area. However, for special embodiments, cameras with lower resolutions might be suitable as well. Especially, the mobile computer device is configured for automatically recognizing the camera resolution.

Especially, a size of the optical sensor of the camera is at least 8.5mm (1/3"), in particular at least 10.2 mm (1/2.5"), preferably at least 14.2 mm (1/1.7"), preferably at least 16.9 mm (2/3"), particularly at least 19.1 mm (1/1.33") or at least 21.2 mm (1/1.2"). Particularly, a size of the optical sensor of the camera is from 8.5 to 25.4 mm (1/3" to 1").

Put differently, a size of the optical sensor of the camera in terms of width × height preferably is at least 4.8 mm × 3.6 mm, in particular at least 5.7 mm × 4.2 mm, preferably at least 7.6 mm × 5.7 mm, preferably at least 8.8 mm × 6.6 mm, particularly at least 9.6 mm × 7.2 mm or at least 10.6 mm × 8.0 mm. Particularly, a size of the optical sensor of the camera in terms of width × height is from 4.8 mm × 3.6 mm to 13.2mm × 8.8 mm.

In general, the larger the sensor size, the more light can be captured by the sensor, which in turn improves the image quality. However, cameras with other sensor sizes might be suitable as well.

Additionally, supplementary lenses might be added to the camera, e.g. for extending or shortening the focal length of the camera.

Also, if available, additional internal wide angle and/or magnification lenses, both optical and/or digital, of the mobile computer device can be accesses on demand for taking the at least one digital image.

The predefined sample area is a two-dimensional sample area.

The two-dimensional sample area preferably is a flat area, especially a flat rectangular area. The two-dimensional sample area is aligned horizontally and/or it is a horizontal sample area.

In the predefined two-dimensional sample area, the solid samples are arranged in a single layer and/or such that there is essentially no overlap of particles in the sample area.

Alternatively or in addition, image processing algorithms that are designed for identifying individual solid particles in particle agglomerates can be used in step c). These special measures will prevent that overlapping of solid particles is biasing the analysis.

Preferably, the predefined two-dimensional sample area, comprises a reference scale and/or has a known size. A reference scale might for example be a character, a geometrical form, a ruler and/or a reference object of known size in the sample area. This allows for a precise determination of the size of the predefined sample area and a precise extraction of particle size parameters in step c).

Preferably, the mobile computer device is configured for automatically determining the size of the predefined two-dimensional sample area, based on the reference scale.

In addition or alternatively, the predefined two-dimensional sample area, has a predefined known size. In this case, preferably, the mobile computer device is configured for setting a predefined size, e.g. from a list of predefined sizes. In this case, no reference scale is required.

Especially, a thin sheet material, preferably of predefined size, is used as the predefined two-dimensional sample area. For example a sheet of paper, e.g. a DIN A5, A4, or A3 paper, is used as the thin sheet material. Also, papers with other formats, such as e.g. tabloid, letter or statement format, can be used. Additionally, a reference scale can be present on the thin sheet material. Preferably, the mobile computer device is configured for automatically determining the size of the predefined two-dimensional sample area.

Papers as predefined sample areas are readily available and rather cheap.

In general, the size of the predefined two-dimensional sample area, affects the minimum identifiable particle size. The smaller the size of the sample area, the smaller solid particles can be identified in the predefined two-dimensional sample area. Thus, for characterizing small particles, small predefined two-dimensional sample areas, are beneficial. Small particles in particular are particles with a particle size D90 not higher than 0.5 mm, preferably not higher than 0.1 mm, especially no higher than 0.063 mm.

Especially, the predefined sample area, in particular the thin sheet material, has a specific color different from the color of the solid particles. This helps to identify individual solid particles in step c) because the grain dimensions and the identification of particles close to the limit of the camera resolution can be identified better. Thus, preferably, the imaging particle analysis comprises a step of subtracting a specific background color.

Especially, the specific color of the predefined two-dimensional sample area, is white. This results in a high contrast when characterizing solid particles typically used in curable compositions, such as e.g. sand particles. However, for other particles, different specific colors of the predefined sample area might be preferable.

Preferably, the predefined two-dimensional sample area, in particular the thin sheet material, is placed on a surface, which in all directions of space is larger than the predefined two-dimensional sample area, and that has a color that is different than the color of the predefined sample area. In particular, the color of the surface is chosen to show a high contrast with regard to the predefined two-dimensional sample area. For example, if the color of the predefined sample area is white, the color of the surface is black.

In these cases, the predefined two-dimensional sample area, is fully surrounded by a frame of a different color. This helps to identify the predefined two-dimensional sample area, in the digital image.

According to a further preferred embodiment, a light emitting luminous surface is used as the predefined two-dimensional sample area. Thereby, the luminous surface in particular is illuminated with a light source such that the luminous surface emits light with a homogeneous light distribution over the whole surface.

Especially, a light table pad, in particular a light pad, is used as the predefined sample area. This is an advantageous possibility to provide a light emitting luminous surface. A light table comprises a flat and luminous surface to be oriented horizontally that is illuminated with a light source from the backside. Especially, the luminous surface consists of a translucent layer that is illuminated from the backside with the light source.

A light pad in particular is a thin light table which a thickness of less than 20% or less than 10% of the width and less than 20% or less than 10% of the length of the luminous surface. Light tables and light pads are known, e.g. in the field of graphics, and commercially available.

When using a light emitting luminous surface, especially a light table, as the sample area, the sample of solid particles is arranged on top of the luminous surface, especially on the frontside of the luminous surface.

Compared to other predefined sample areas, such as e.g. papers, luminous surfaces, especially light tables or light pads, are in particular beneficial since shadows of the sample particles can be omitted, the contrast can be increased, less artefacts are produced, better particle recognition, especially of bright particles and/or small particles, is possible, and the fit of calculated outlines can be improved which in turn increases accuracy of particle shape parameter and particle size determination. Overall, the accuracy of the results can be improved.

Especially, the luminous surface is illuminated such that it emits a color different than the color of the solid particles, preferably the luminous surface is illuminated such that it emits white light.

In particular, the light source comprises a source of withe light. Optionally the light source additionally comprises a source of light of a color different from white. In particular, the color of the light source is switchable between the different colors. Colors different than white enhance detection of whitish, bright sands.

In particular, the light source is an LED light source. Compared to other light sources, this allows for minimizing heat evolution on the translucent surface or in the sample area, respectively. This reduces the risk of thermally induced changes of the sample.

LED may cause temporal light modulation disturbances. Temporal light modulation is a change in the luminous quantity or spectral distribution of light over time. Such modulations may result in undesirable visual perceptions such as flickering, stroboscopic effects and phantom array effects. Such effects are also known as temporal light artefacts. Such effects are described for example by J.A. Veitch et al in "On the state of knowledge concerning the effects of temporal light modulation" 20 Lighting Res. Technol. 2021, 53, 89-92. It is preferable within the present context, to avoid such temporal light modulation and effects resulting therefrom. Thus, according to some embodiments, the light source is configured to avoid effects resulting from temporal light modulation.

Preferably, the light emitting luminous surface, especially a light table or a light pad, is configured such that the light intensity of the luminous surface can be adjusted, especially continuously or in discrete steps. For example, the light emitting luminous surface, especially the light table or the light pad, is configured such that the light intensity can be switched between 2 - 5, especially 3 - 4, different light intensities.

In a further preferred embodiment, the light emitting luminous surface, especially the light table or the light pad, is configured such that it emits polarized light. This can e.g. be achieved by using a light source producing polarized light and/or a polarization filter arranged behind, on top of and/or within the luminous surface. A polarization filter can e.g. be selected from a foil. Polarized light can be used to further enhance the determination of the particle parameters and shapes. A foil can also be a colored foil.

In further preferred embodiments, the emitted light is such that it does not cause interferences.

Furthermore, the luminous surface, especially of a light table or a light pad can, be covered with a protective foil, e.g. for increasing scratch resistance, whereby preferably the protective foil is transparent with respect to the emitted light of the luminous surface. In particular, the protective foil is made of synthetic material. Especially, the protective foil is a replaceable foil.

Particularly, the light emitting luminous surface, especially the light table or the light pad, comprises a frame surrounding the light emitting luminous surface, whereby, the frame has a color different than the light emitting luminous surface, especially a darker color than the light emitting luminous surface, in particular a black color. This helps to identify the predefined two-dimensional sample area, in the digital image.

For example, as size of the light emitting luminous surface, especially of the light table or the light pad, is equal to the size of a DIN A5, A4, or A3 paper or has the size of the tabloid, letter or statement format. Typically, light pads are slightly bigger in size than any of the afore mentioned formats to ensure that a paper of a given format would fit perfectly to a light pad of such format. Thus, the actual size of the light emitting luminous surface, especially of the light table or the light pad, may also be slightly bigger than any of the afore mentioned formats. Preferably, the mobile computer device is configured for manually and/or automatically determining the size of the light emitting luminous surface.

Taking the at least one digital image of the sample of solid particles with the camera preferably is performed under daylight conditions and/or with a light source, e.g. a flashlight, for illuminating the solid particles. Thereby, the light used for illuminating the solid particles preferably is well dispersed in order to avoid shadows and light inhomogeneities. The mobile computer device preferably is configured for automatically adjusting light conditions in order to obtain a balanced exposure.

In a further preferred embodiment, the camera is aligned plane-parallel, especially horizontally, to the predefined two-dimensional sample area. Preferably, the mobile computer device is configured for automatically warning the user and/or for preventing taking the at least one image as long as there is a non-plane-parallel alignment. In this case, the mobile computer device preferably comprises at least one position sensor which can be assessed when performing the inventive method.

Especially, the camera is aligned horizontally, in particular horizontally and plane-parallel to the predefined sample area, when taking the at least one digital image of the sample of solid particles in step b). Thereby, preferably, the predefined sample area is a horizontal area and/or it is aligned horizontally. Horizontal alignment can be conducted manually or by using a supporting function that are known to the skilled person.

Especially, the camera is aligned horizontally if its optical axis runs vertically. The optical axis is an imaginary line that defines the path along which light propagates through the camera system.

Taking the at least one digital image of the sample of solid particles in step b) with the camera in horizontal alignment, in particular horizontally and plane-parallel to the predefined sample area, greatly simplifies the image capturing process. Specifically, by adjusting the height of the camera over the predefined sample area, a share of the sample area in the image can be easily maximized with the horizontal alignment.

Also, a horizontal alignment of the predefined sample area or a horizontal sample area is beneficial since the solid particles will automatically remain stable and motionless in their position during the image capturing process. Thereby, the predefined sample area can for example be located on a table or any other essentially horizontal surface.

With the camera in vertical alignment and a non-horizontal alignment of the sample area, this is less convenient and requires special measures to keep the solid particles of the sample in place.

However, the method can be implemented as well with a non-plane-parallel alignment of the camera.

In particular, when taking the at least one digital image of the sample of solid particles with a camera of a mobile computer device in step b), the solid particles of the sample remain motionless. This significantly improves the image quality and the imaging particle analysis in step c).

In a further preferred embodiment, at least two consecutive digital images of the sample area are taken. In this case, preferably, step c) is performed with the at least two digital images. This can be helpful for increasing the number of statistical counts and for more precisely determining the at least one particle size parameter and at least one particle shape parameter. In particular, in step c), the at least two digital images are superimposed.

Especially, when taking the at least one image, the camera is aligned so that a share of the sample area in the image is maximized. This can be implemented for example by providing alignment instructions to the user and/or by automatically adjusting at least one setting of the camera, e.g. the focal length of the camera. Thus, preferably, the mobile computer device is configured accordingly.

Highly preferred, a minimum detectable particle size is calculated, preferably by taking into account the resolution of the camera, the area share of the sample area in the total area of the image, and the real size of the sample area. Preferably, the mobile computer device is configured accordingly.

Especially, if the minimum detectable particle size is below a predetermined threshold, a warning is provided to the user, alignment instructions are provided to the user and/or a setting of the camera, e.g. the focal distance, is adjusted. The predetermined threshold can e.g. be set manually. This helps to avoid taking images under unsuitable conditions.

The imaging particle analysis of the at least one digital image in step c) can be implemented with known and readily available image analysis algorithms, e.g. by using software packages and/or libraries provided in Matlab (by MathWorks^{®}), OpenCV (cf. https://opencv.org), ImageJ (by Wayne Rasband; cf. https://imagej.net) and/or artificial intelligence algorithms.

The at least one particle size parameter extracted preferably comprises at least one of the following parameters:
- the particle size distribution of the population of particles identified in the at least one digital image;
- at least one statistical parameter selected from the group of average particle size, mean diameter, and/or Dₓ-value with x = 1 - 100, especially D₁₀, D₅₀, D₈₅ and D₁₀₀ values; and/or
- a deviation from a predefined nominal value and/or nominal distribution, e.g. from fuller curves, standard sieving curves for specific concrete types; and/or
- the fineness modulus.

According to especially preferred embodiments, the at least one particle size parameter extracted comprises or is the particle size distribution of the population of particles identified in the at least one digital image.

These are highly relevant parameters when providing formulations of curable compositions with solid particles. However, other parameters might be provided as well.

Thereby, the extracted particle size distribution in particular is meant to correspond to the particle size distribution as defined in standard EN 933-1:2012.

Another possible way to determine particle size distribution is laser light diffraction as described in ISO 13320:2009. Thus, the extracted particle size distribution in especially is meant to correspond to the particle size distribution as defined in standard ISO 13320:2009.

A Dₓ value has the meaning that a proportion of the given assembly of particles of x% has a lower particle size than the given value. Thus, a D₉₀ value, for example, means that 90% of the assembly of particles has a particle size smaller than the D₉₀ value given. Accordingly, the average particle size corresponds in particular to the D₅₀ value (50% of the particles are smaller than the given value, 50% are correspondingly bigger).

In general, the at least one particle size parameter extracted preferably comprises at least the particle size distribution of the population of particles identified in the at least one digital image.

Especially, for particle sizes below the minimum detectable particle size, the particle size distribution may be extrapolated based on the extracted particle size distribution. For example, polynomial extrapolation is used, especially based on Lagrange interpolation or using Newton's method of finite differences to create a Newton series that fits the extracted particle size distribution.

Especially, the at least one particle shape parameter extracted comprises at least one of the following parameters:
- roundness,
- sphericity,
- aspect ratio,
- roughness,
- solidity,
- flakiness index,
- shape index,
- percentage of crushed and broken surfaces, and/or
- angularity.

These are parameters that so far are hardly analyzed in daily work. However, they have significant influence for optimizing the grading curve of a curable composition.

Especially, the particle shape parameters are meant to correspond to the shape parameters as defined in standards EN 933-1:2012 to -7:2012.

Especially, the particle shape parameters include the aspect ratio expressed as the maximum Feret diameter to the minimum Feret diameter.

Further shape parameters which can be extracted are given in Blott and Pye, Sedimentology (2008) 55, 31-63.

In particular, the at least one particle shape parameter is extracted with regard to particles of a predetermined size only, especially to particles larger than a given threshold size. For relatively small particles, the particle shape of solid particles affects the properties of curable compositions less. For example, the at least one particle shape parameter is extracted with regard to particles > 0.5 mm, in particular > 1 mm.

Nevertheless, it is possible to analyze the shape parameters of all solid particles if desired.

Furthermore, it is possible to extract the at least one particle shape parameter with regard to particles larger than a given lower threshold size and particles smaller than a given upper threshold size. Thereby, in particular, at least two, at least three or more different particle shape parameters can be extracted simultaneously within the range between the lower threshold and the upper threshold.

This allows for identifying shape parameters of particles with specific sizes, which are for example known to affect certain properties of interest of curable compositions, such as e.g. the rheology of a curable composition.

According to another preferred implementation, for each of at least two or more predefined size fractions, e.g. sieve size fractions, of the particles, at least one individual particle shape parameter is extracted. Thereby, especially, at least one individual mean value of the at least one particle shape parameter is extracted for each size fraction. This allows for providing detailed information about the size dependent distribution of the particle shape parameters.

For example, a mean value of the at least one particle shape parameter is provided for each of the least two or more predefined particle size fractions, e.g. sieve size fractions, separately. Optionally, the particle counts per size fraction can be normalized by the volume of the particles considered, e.g. in order to obtain data comparable to particle size distributions. In addition, in this case, at least two, at least three or more different particle shape parameters can be provided simultaneously.

Such data can e.g. be presented in a bar plot with the predefined particle size fractions as categories and the corresponding mean values of the at least one particle shape parameters in the form of bars with heights or lengths proportional to the values that they represent.

Furthermore, it is possible to provide the mean value of the at least one particle shape parameter with regard to particles in several selected size fractions, e.g. particles being present in size fractions above and/or below a given fraction threshold. Similar to the above, this allows for identifying shape parameters of particles in size fractions, which are for example known to affect certain properties of interest of curable compositions.

In a further preferred implementation, for each of the at least one digital image, an outline image is generated. An outline image is also called a contour image and comprises the outlines of all of the identified particles in the digital image. The outline image can be made available in step d) via a user interface, via a machine interface and/or on a data storage medium. For example, the outline image can be displayed within the application and/or stored on an external server. The outline image can serve as tool to evaluate the quality of the digital image and/or the analysis performed.

Especially, in step b), at least two, preferably at least three, in particular at least five or at least ten, digital images are taken and for each image a particle analysis is performed in step c), and by taking into account each of the at least one particle size parameter, and optionally the at least one particle shape parameter, individually extracted from the at least two images, a deviation, especially the standard deviation, of the at least one particle size parameter, and optionally the at least one particle shape parameter is determined. The deviation can serve as tool to evaluate the quality of the digital images and/or the analysis performed.

Especially, if a deviation is above a predetermined threshold, a warning can be provided to the user and/or if a deviation is above a predetermined threshold, a digital image and/or an outline image giving rise to diverging parameters might be identified and/or indicated.

Preferably, the inventive method further comprises the step of assigning at least one attribute of the sample of solid particles. Especially, the attribute is selected from:
- a unique identifier of the sample of solid particles;
- a chemical and/or physical property of the sample of solid particles, especially
   ∘ the nature of the solid particles, e.g. sand, limestone and/or polymeric particles;
   ∘ the type of the solid particles, e.g. natural, crushed, manufactured, recycled and/or re-used solid particles;
   ∘ a maximum grain size of the solid particles;
   ∘ a minimum grain size of the solid particles;
   ∘ the state of the solid particles, e.g. wet or dry; and/or
   ∘ pretreatments received, e.g. untreated or washed.
- descriptive information of the sample of solid particles, especially
   ∘ the location of the source of the solid particles, e.g. manually or automatically by position sensors;
   ∘ the intended use, e.g. a project name and/or a customer name; and/or
   ∘ a general comment.

Especially, the "manufactured" particles are meant to be aggregates as defined e.g. in the Annual review 2018-2019 of the European Aggregates Association, on page 5.

Preferably, the mobile computer device is configured to query the at least one attribute of the sample of solid particles attributes, especially before, during and/or after steps a) to b).

Especially, a unique identifier of the sample of solid particles is generated automatically.

In particular, the method is at least partly, especially completely, performed on the mobile computer device.

In case the method is completely performed on the mobile computer device, the complete characterization can take place on the mobile computer device without requiring any communication network. Also, the at least one digital image, preferably together with the at least one attribute can be stored on the mobile computer device, e.g. for sharing, recalling and/or further evaluation.

Preferably, the mobile computer device is configured such that the at least one digital image, preferably together with the at least one attribute can be transferred to an external device via communication means, e.g. a communication interface of the mobile device, for storing. Likewise, the mobile computer device preferably is configured for recalling the stored data from the external device. According to another preferred implementation, the image analysis in step c) and/or the making available in step d) is/are conducted on a separate computer device, e.g. on a server.

In this case, preferably, the at least one digital image, preferably together with the at least one attribute is transferred to the external device via communication means, e.g. a communication interface of the mobile device. Such a decentralized solution is beneficial since computationally intensive step c) can be performed on another device which in turn helps to increase runtime of the mobile computer device. Furthermore, steps c) and/or d) can be optimized by updating the software part on the side of the external device without the user having to update the software part on the mobile computer device.

Thereby, if there is no communication network available, the at least one digital image, preferably together with the at least one attribute, can be stored temporarily on the mobile computer device and transferred to the external device later on when the communication network is available.

In this case, the image, preferably together with the at least one attribute, is stored on an external computer device, e.g. a server, in particular for sharing, recalling and/or further evaluation.

In step d) the at least one particle size parameter and the at least one particle shape parameter are made available via a user interface, via a machine interface and/or on a data storage medium. If desired, the at least one digital image, optionally together with the at least one attribute, can be made available in addition. However, this is not a requirement since for daily work the image as such is hardly important for a user.

If the data is made available via a user interface, this can be done directly on the mobile computer device and/or an external computer device. Thereby, for example the at least one particle size parameter and the at least one particle shape parameter are plotted in a graph, e.g. the particle size distribution, and/or presented in an animated plot, e.g. in a roundness versus sphericity plot or the bar plot of mean particle size parameter versus sieve opening.

Making available the data via a machine interface allows for transferring the data to an external computer device, e.g. a server and/or a desktop computer.

Also it is possible to make available the data on a data storage medium, e.g. on an internal data storage medium of the mobile computer device, a storage device attached to the mobile computer device and/or on a storage device of an external computer.

Especially, the at least one particle size parameter and the at least one particle shape parameter, optionally together with the at least one attribute, and optionally with the at least one image, are written in a data file with predefined file format. E.g. the file format is chosen from json, csv, txt and/or pdf. However, other file formats can be used as well. Typically, the data file does not include the at least one image. This helps to reduce the file size.

In particular, the data file is transferred to another application, a further mobile computer device and/or an external computer device. Transfer can be effected by any kind of communication means, e.g. wireless communication and/or wired communication. This allows a user to share the characterization of the solid particles with other users, transfer it to another application for further evaluation and/or to a data storage server. Thus, preferably, the mobile computer device is configured for transferring a data file to another application, a further mobile computer device and/or an external computer device.

A size of the solid particles for example ranges from >0 to 125 mm, preferably from 10 µm to 32 mm, more preferably from 0.063 mm to 16 mm, especially from 0.1 mm to 2 mm. Such kind of particles are typically used in curable compositions. However, the method can be used for characterization of other particles as well.

In particular, the solid particles of the sample are selected from sand, aggregates, fibers and/or glass spheres. However, other solid particles can be used as well, especially sand replacements, manufactured sands, crushed and/or recycled construction materials, bio aggregates, natural or synthetic fibers and/or polymeric particles.

Further advantageous configurations of the invention are evident from the exemplary embodiments.

### Brief description of the drawings

The drawings used to explain the embodiments show:
- Fig. 1: A flow chart of an inventive computer-implemented method;
- Fig. 2: A schematic overview of a system comprising means for carrying out the method of Fig. 1;
- Fig. 3: A schematic view of the second step of the method of Fig. 1 whereby a user (not shown) holding a smartphone is taking an image of a sample of solid particles of different sizes;
- Fig. 4: An example of the structure of a data file;
- Fig. 5: A flow chart of another computer-implemented method;
- Fig. 6: A bar plot of selected particle shape parameters (roundness, sphericity and aspect ratio) per particle sieve size fraction;
- Fig. 7: An outline image overlaid over a digital image;
- Fig. 8: A schematic view of a light pad;
- Fig. 9: A comparison between a sample of particles on the non-illuminated surface of the light pad of Fig. 8 (left side) and the same sample of particles on the illuminated surface of the light pad of Fig. 8 (right side);
- Fig. 10: Another bar plot of selected particle shape parameters (roundness/R, sphericity/SP and aspect ratio/AR) per particle sieve size fraction together with the corresponding particle count per particle sieve size fractions with sizes > 0.5 mm.

### Exemplary embodiments

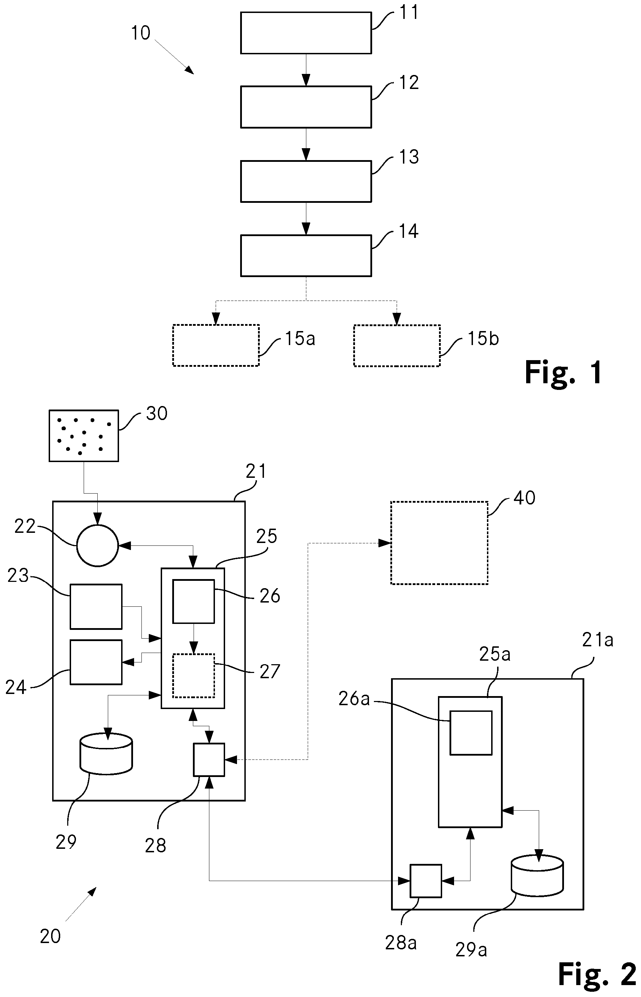

Fig. 1 shows a flow chart of an inventive computer-implemented method 10. In a first step 11, a sample of solid particles to be analyzed, e.g. a sand sample with particle sizes ranging from >0 to 2 mm, is provided on a two-dimensional sample area of known size. The sample area is for example formed by a sheet of paper of A4 size.

In a second step 12, a digital image of the sample of solid particles is taken with a camera of a mobile computer device, e.g. a smartphone. The camera for example has a 4K resolution.

Thereafter, in a third step 13, an imaging particle analysis of the digital image is performed for extracting at least one particle size parameter, e.g. the particle size distribution, and/or at least one particle shape parameter, e.g. roundness or sphericity, of the population of particles identified in the at least one digital image.

In a fourth step 14, the at least one particle size parameter and the at least one particle shape parameter are made available via a user interface, e.g. a display of the mobile computer device.

In an optional fifth step 15a, a formulation of a curable composition comprising at least a binder and solid particles is provided, whereby nature and/or proportion of at least one component of the formulation, especially an additive, e.g. a plasticizer, in the formulation, is determined by taking into account the at least one particle size parameter and the at least one particle shape parameter.

Alternatively or in addition, in an optional step 15b, a curable composition comprising at least a binder and solid particles is provided,
whereby step 15b comprises mixing the solid particles with the binder and any optional further component, whereby, the at least one particle size parameter and the at least one particle shape parameter are considered for determining nature and/or proportion of at least one of the components, especially of an additive, in the composition.

Fig. 2 shows a schematic overview of a system 20 (not claimed) comprising means for carrying out the method shown in Fig. 1.

Specifically, the system 20 comprises a smartphone 21 with a camera 22, a touch sensitive display comprising input device 23 and a display 24, a data processing unit 25 with a random access memory, a data storage device 29 and a wireless communication interface 28.

In operation, an application 26 is executed in the data processing unit 25, whereby the application is configured for performing steps 12 and 14 of the method described with Fig. 1.

Specifically, the application 26 assists a user in taking an image of solid particles in a two-dimensional sample area 30 with the built-in smartphone camera 22. Thereby, the application is for example configured for automatically warning the user and/or for preventing taking the image as long as there is a non-plane-parallel alignment. This can be achieved by assessing the position sensors of the smartphone (not shown). Also, the application is configured for automatically adjusting light conditions in order to obtain a balanced exposure. The digital image taken is stored in the random access memory and/or the data storage 29.

Additionally, the application 26 asks the user to enter one or more attributes of the sample via the input device and assigns a unique identifier to the sample. Attributes are e.g. the maximum grain size of the solid particles, the type of the solid particles (natural, crushed, manufactured, recycled; re-used solid particles), the state of the solid particles (wet or dry), the pretreatment (washed or untreated), the location of the source of the solid particles, the intended use (project name, customer name); and/or a general comment.

The query can e.g. be made by presenting to the user input fields, selection fields, maps and/or text input fields on the display 24 and storing the data provided by the user via the input device 23 together with the at least digital image in the random access memory and/or the data storage 29.

For example, the location of the source of particles can be provided manually by the user, e.g. by entering geo coordinates in input fields and/or by marking the position on a map shown on the display 24. It is however possible to automatically provide the location of the source of the solid particles e.g. by sensors of a global navigation satellite system, such as e.g. GPS, Galileo, Beidou and/or Glonass sensors. Thereby, the user might be requested to confirm the automatically determined position.

In the system 20 shown in Fig. 2, step 13 of the method shown in Fig. 1 is performed on an external server 21a. Specifically, the image taken with the smartphone camera 22, optionally together with the attributes, is transferred via the wireless communication interface 28 (or any other communication interface) and a network (e.g. the internet; not shown) to the server 21a. The server 21a receives the data via its communication interface 28a and forwards it to an imaging particle analysis application 26a running in a processing unit 25a.

The image, optionally together with the attributes can be stored on a data storage 29a of the server 21a for later sharing, recalling and/or further evaluation.

The application 26a performs an imaging particle analysis of the digital image whereby at least one particle size parameter, e.g. the particle size distribution, and at least one particle shape parameter, e.g. roundness or sphericity, of the population of particles identified in the at least one digital image is extracted. Thereby one or more attributes may be considered in the analysis as well.

The application 26a is implemented for example with image analysis algorithms such as e.g. software packages and/or libraries provided in Matlab, OpenCV and/or ImageJ, and/or with artificial intelligence software.

After the image analysis is finished, the at least one particle size parameter, e.g. the particle size distribution, and at least one particle shape parameter, e.g. roundness or sphericity, are sent back to the smartphone 21 or the application 25 being executed on it, respectively, via the communication interfaces 28a, 28.

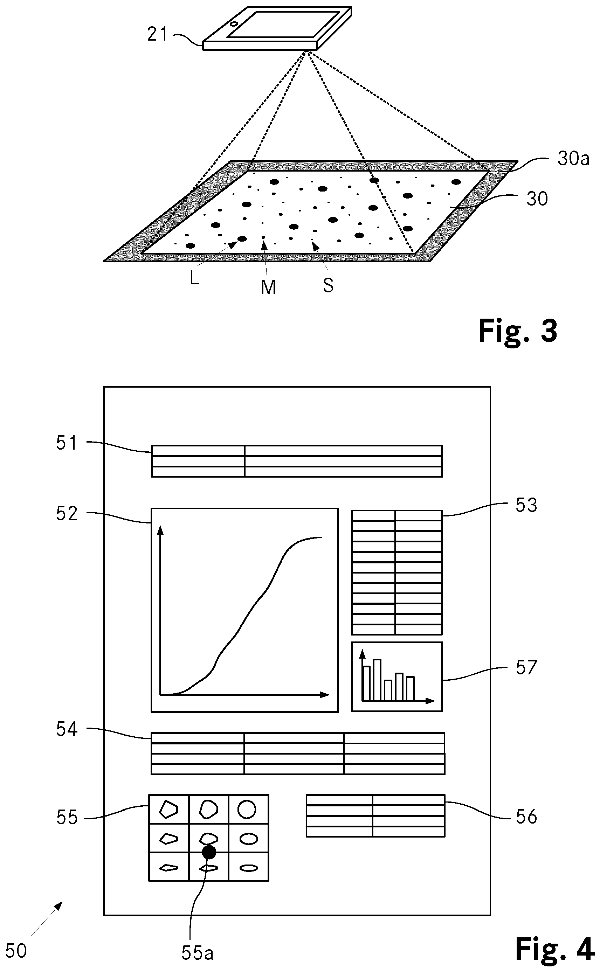

The application 25a then makes available the at least one particle size parameter and/or the at least one particle shape parameter via the display 24, or saves the parameters on the data storage 29, preferably together with the attributes, for later sharing, recalling and/or further evaluation. This data can be stored for example in the form of a data file having a file format chosen from json, csv, txt, pdf, and/or a proprietary file format. Especially, a file format capable of being read by the application called "Sika Mix Design App" and/or any other additional application is chosen. See Fig. 4 for an example.

Additionally, the application 25 is configured for sharing the at least one particle size parameter and the at least one particle shape parameter and the attributes with another user by sending them, in particular as a data file, via the communication interface 28 to a further computer device 40, e.g. a smartphone of another user. This can for example be initiated by the user via input device 23, e.g. by pressing a button shown on the display 24.

Furthermore, the system 20 may comprise an optional mix design application 27, which can be executed with the processing unit 25. The mix design application is configured for providing a formulation of a curable composition comprising at least a binder and solid particles is provided, whereby nature and/or proportion of at least one component of the formulation, especially an additive, e.g. a plasticizer, in the formulation, is determined by taking into account the at least one particle size parameter and the at least one particle shape parameter obtained with the first application 26.

Thus, for example, the first application can forward the at least one particle size parameter and the at least one particle shape parameter, and optionally the attributes, to the mix design application 27 via an appropriate application interface. Also the data to be sent to the mix design application 27 can be provided and transferred to the mix design application 27 in the form of a data file. In addition or alternatively, the mix design application can be configured for loading this data from the data storage 29 and/or in the form of a data file.

The mix design application 27 is configured for making available the formulation provided on the display 24, sending it via the communication interface 28 to another computer system, e.g. server 21a, or to a further smartphone 40, and/or save it on the data storage 29 for later sharing, recalling and/or further evaluation.

Fig. 3 shows a schematic view of step 12 of the method shown in Fig. 1. Thereby, a user (not shown), holding a smartphone 21 in his hands, takes an image of a sample of solid particles L (large), M (medium), S (small) of different sizes, e.g. sand particles with particle sizes ranging from >0 to 2 mm, being provided on a white sheet of paper of A4 size serving as a two-dimensional sample area 30. The sheet of paper is placed on a surface with high contrast, e.g. black surface, 30a that in all directions of space is larger than the two-dimensional sample area 30. Thus, the two-dimensional sample area 30 is fully surrounded by a frame of a different color.

Fig. 4 shows an example of the structure of a data file 50 in pdf file format. The data file 50 comprises a table 51 with attributes provided by the user, e.g. the type of the solid particles (natural, crushed, manufactured, recycled; re-used solid particles), the state of the solid particles (wet or dry), the pretreatment (washed or untreated), the location of the source of the solid particles, the intended use (project name, customer name); and/or a general comment.

Also, the file 50 comprises a graph 52 representing the particle size distribution, a table 53 comprising the calculated sieve size pass rate of the solid particles and/or the calculated proportion of retained solid particles, and a table 54 with statistical parameters, such as D₁₀, D₅₀, D₈₅, and D₁₀₀ values as well as the fineness modulus of the solid particles analyzed.

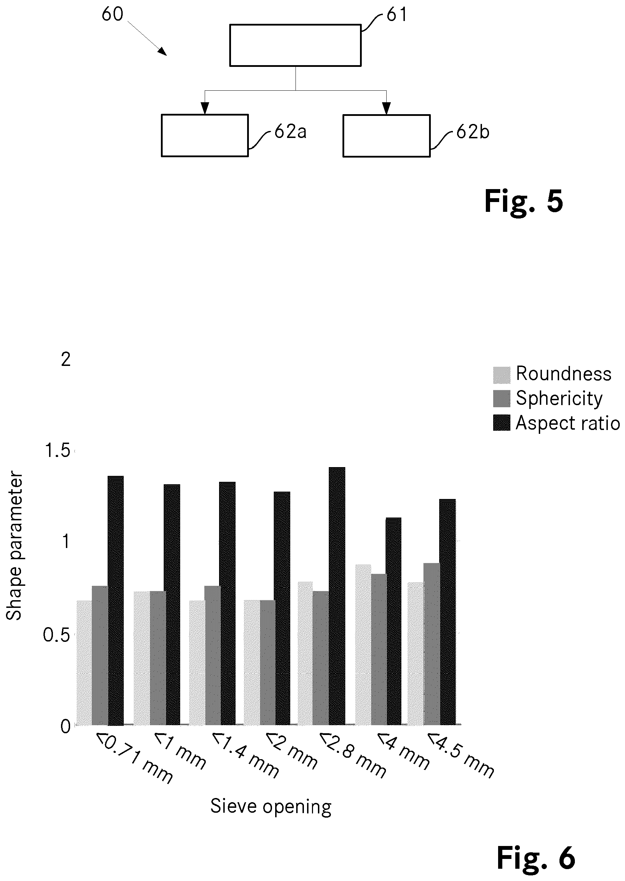

Furthermore, the file 50 comprises a two-dimensional plot 55 indicating the mean particle shape (for example roundness or sphericity) with a marker 55a. Additionally, the file 50 comprises a bar plot 57 displaying the mean value of selected particle shape parameters (for example roundness, sphericity and aspect ratio) per particle sieve size fraction. A more detailed view of the bar plot 57 is shown in Fig. 6.

Fig. 5 shows a flow chart of another computer-implemented method 60 (not claimed). Thereby, the particle size distribution and at least one particle shape parameter of a sample of solid particles are provided in a first step 61. These parameters can be obtained with the method shown in Fig. 1 or with any other method, e.g. manually.

Subsequently, in next step 62a, a formulation of a curable composition comprising at least a binder and solid particles is provided, whereby nature and/or proportion of at least one component of the formulation, especially an additive, e.g. a plasticizer, in the formulation, is determined by taking into account the particle size distribution and the at least one particle shape parameter.

This can be achieved with a mix design application as described above, i.e. mix design application 27. This application is configured for making available the formulation provided on the display 24, sending it via the communication interface 28 to another computer system, e.g. server 21a, or to a further smartphone 40, and/or save it on the data storage 29 for later sharing, recalling and/or further evaluation.

Alternatively or in addition, in an optional step 62b, a curable composition comprising at least a binder and solid particles is provided, whereby step 62b comprises mixing the solid particles with the binder and any optional further component, whereby, the at least one particle size parameter and/or the at least one particle shape parameter are considered for determining nature and/or proportion of at least one of the components, especially of an additive, in the composition.

As a representative example, a curable mortar composition comprising cement (CEM I), water, a plasticizer (Sika^{®} Viscocrete^{®} 111 P) and silica sand (D85-value = 2.36 mm) was produced as follows:
1. The particle size distribution as well as the sphericity, roundness and aspect ratio (= particle shape parameters) of the sand used were determined according to the method shown in Fig. 1.
2. The particle size distribution as well as the particle shape parameters were transferred to the "Sika Mix Design App" (available from Sika Services AG) for calculation of a mortar composition with a predefined slump flow of 370 mm (according to EN 12350-5:2019). Thereby, the proportions of cement, water, plasticizer and silica sand were adjusted to achieve the target slump flow.
3. Subsequently, the mortar composition as calculated by the "Sika Mix Design App" was produced by mixing the respective proportions of cement, water, plasticizer and silica sand. Then the slump flow of the so produced mortar composition was determined (according to EN 12350-5:2019) in order to compare the real slump flow of the mortar composition produced with the target slump flow of 370 mm.
4. For reasons of comparison, steps 2 and 3 were repeated without considering the particle shape parameters (sphericity, roundness and aspect ratio) in the "Sika Mix Design App" but otherwise identical conditions. Thus, in this case only the particle size distribution was considered in the "Sika Mix Design App" when calculating the mortar composition.

The results were as follows:

| | **Measured slump flow** | **Deviation from Target slump flow** |
|---|---|---|
| Target slump flow | 370 mm | - |
| Slump flow of composition calculated with particle size and particle shape parameters | 374 mm | 4 mm (+1.1%) |
| Slump flow of composition calculated without particle shape parameters | 346 mm | -24 mm (-6.5%) |

Thus, when considering the shape parameters, it is possible to predict a mortar composition that has a slump flow very close to the desired target value. Without the shape parameters, the deviation from the target slum flow is significantly higher.

Similar tests have been performed with other sands (not shown here). A statistical evaluation of the data has shown that considering the particle shape parameters for calculating a mortar composition having a desired target slump flow is highly relevant in general and results in significantly better predictions when compared with calculations that are not taking into account the shape parameters.

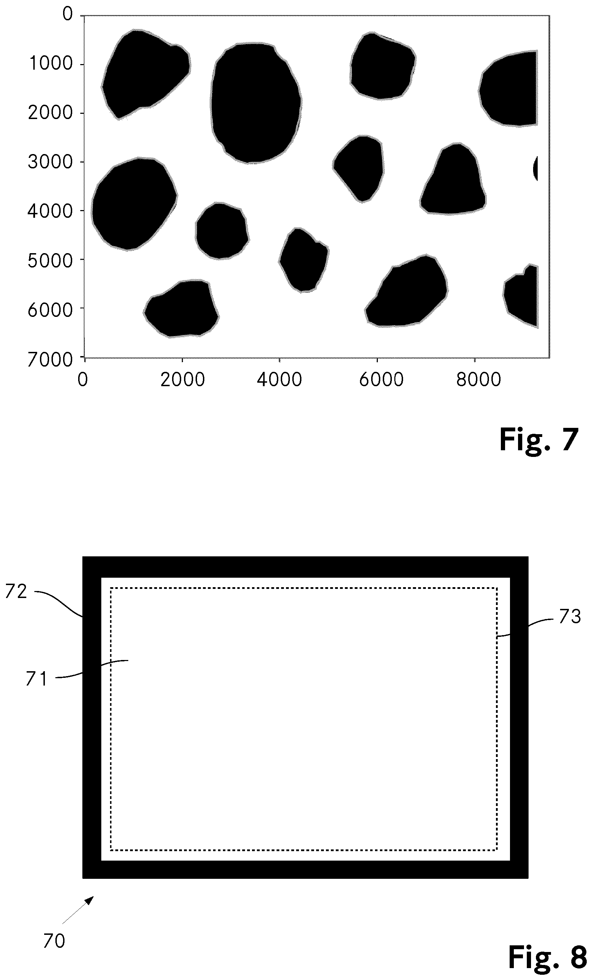

Fig. 7 shows an outline plot overlaid over the corresponding digital image. The outline plot can be used in addition to check the quality of the digital image and/or the analysis performed.

Fig. 8 shows a schematic view of a light pad 70 which can be used as the two dimensional sample area 30 instead of the sheet of paper used in the setup of Fig. 3. The light pad comprises of a light emitting luminous surface 71 made of a translucent layer that is illuminated with a white LED light source 73 (indicated by a dashed rectangle) from the backside. The light emitting luminous surface 71 has a size of a DIN A4 paper and is fully surrounded by a black frame 72.

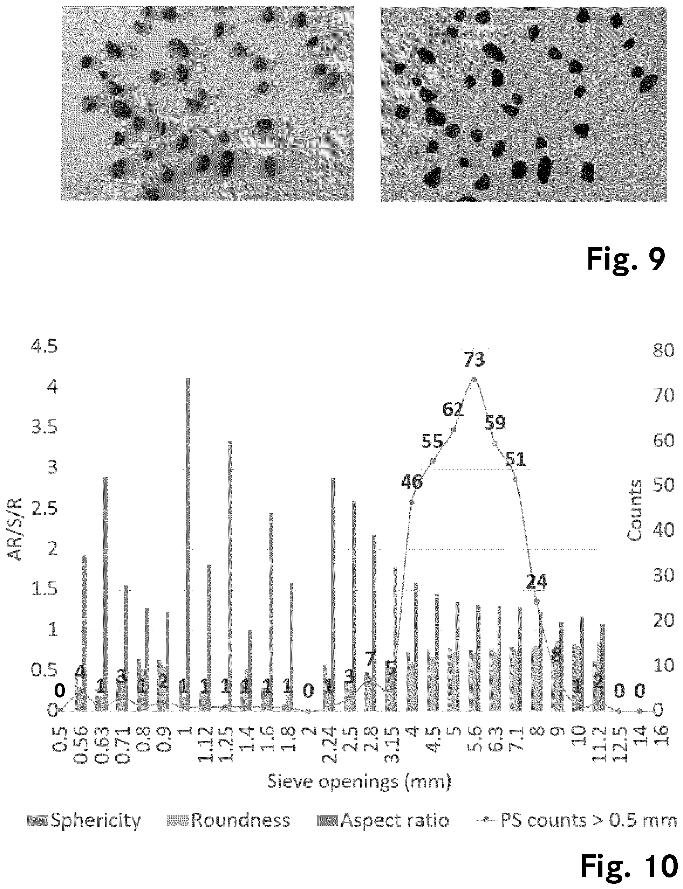

Fig. 9 shows a sample of particles on the non-illuminated surface of the light pad 70 of Fig. 8 (left side) and the same sample of particles on the illuminated surface of the light pad 70 of Fig. 8 (right side). As evident from the comparison, the contrast on the right side is clearly better and there are no shadows visible.

Fig. 10 shows an example of the particle counts of a sample of sand particles with a particle size > 0.5 mm per particle sieve size fractions (right axis), and additionally the corresponding mean values of three different particle shape parameters (roundness/R, sphericity/SP and aspect ratio/AR) per particle sieve size fraction (left axis).

The data shown in Fig. 10 was obtained with the method and the system described in Fig. 1 and 2. The application 26 used in this method may be further configured to calculate an overall mean value of the particle shape parameters for a selectable range of particle sieve size fractions, e.g. from a lower threshold of 4 mm to an upper threshold of 8 mm or alternatively from a lower threshold of 1 mm up the maximum sieve size fraction of 16 mm.

It will be appreciated by those skilled in the art that the present invention can be implemented in other specific forms. The presently disclosed implementations and embodiments are therefore considered in all respects to be illustrative and not restricted.

For example, instead of using server 21a, the application 26 can be configured as a standalone application capable of executing all of the steps 11, 12, 13, 14 and optionally 15a of the method of Fig. 1.

Likewise, it is possible to omit optional functions of the system 20 (not claimed), e.g. sharing of data with other computer devices, or adding additional functions, such as for example automatically retrieving of positional data via positioning sensors.

The method can as well be used for characterizing solid particles other than sands.

Also, it is possible to provide system 20 (not claimed) with mix design application 27 but without application 26. Such a system is for example suitable for performing the method as shown in Fig. 5 (not claimed). Thereby, the at least one particle size parameter and the at least one particle shape parameter can be inputted manually and/or being read from the data storage 29 and/or any other data storage, e.g. data storage 29a of server 21.

## Claims

1. Computer-implemented method for characterization of solid particles, especially sand particles, comprising the steps of:
a) Providing a sample of solid particles to be analyzed in a predefined sample area;
b) Taking at least one digital image of the sample of solid particles with a camera of a mobile computer device;
c) Performing an imaging particle analysis of the at least one digital image for extracting at least one particle size parameter, and optionally at least one particle shape parameter, of the population of particles identified in the at least one digital image;
d) Making available the at least one particle size parameter, and optionally the at least one particle shape parameter, via a user interface, via a machine interface and/or on a data storage medium,
**characterized in that** in step a) the sample area is a two-dimensional sample area which is aligned horizontally and wherein the solid particles are arranged in a single layer and/or such that there is essentially no overlap of particles in the sample area.

2. Method according to claim 1, whereby the mobile computer device comprises a human interface device, especially comprising an input device and a display, and, preferably a wireless communication interface.

3. Method according to any of preceding claims, whereby the mobile computer device is selected from a mobile phone, a mobile computer or a portable computer, and/or a head-mounted display with camera.

4. Method according to any of preceding claims, whereby the camera is a camera for taking images in the visible spectrum, especially color images.

5. Method according to any of preceding claims, whereby the camera has a resolution of at least 2 megapixels, especially at least 5 megapixels, preferably at least 8 megapixels, particularly at least 12 megapixels, highly preferred at least 20 megapixels, even more preferred at least 50 megapixels or at least more than 100 megapixels.

6. Method according to any of preceding claims, whereby the sample area comprises a reference scale and/or has a known size.

7. Method according to any of preceding claims, whereby the predefined sample area is a thin sheet material, especially of predefined size.

8. Method according to any of preceding claims, whereby the particles of the sample are selected from sand, aggregates, fibers and/or glass spheres.

9. Method according to any of preceding claims, whereby when taking the image, the camera is aligned so that a share of the sample area in the image is maximized, especially by providing alignment instructions to the user and/or by automatically adjusting at least one setting of the camera, e.g. the focal length of the camera.

10. Method according to any of preceding claims, whereby a minimum detectable particle size is calculated by taking into account the resolution of the camera, the length share of the sample area in the total area of the image, and the real length of the sample area.

11. Method according to any of preceding claims, whereby, if a minimum detectable particle size is below a predetermined threshold, a warning is provided to the user, alignment instructions are provided to the user and/or a setting of the camera, e.g. the focal distance, is adjusted automatically.

12. Method according to any of preceding claims, whereby the at least one particle size parameter extracted comprises the particle size distribution of the population of particles identified in the at least one digital image.

13. Method according to any of preceding claims, whereby for each of the at least one digital image, an outline image is generated, and, preferably, the outline image is made available in step d) via a user interface, via a machine interface and/or on a data storage medium.

14. Method according to any of preceding claims, whereby in step b), at least two, preferably at least three, in particular at least five or at least ten, digital images are taken and for each image an imaging particle analysis is performed in step c), and by taking into account each of the at least one particle size parameter, and optionally the at least one particle shape parameter, individually extracted from the at least two images, a deviation, especially the standard deviation, of the at least one particle size parameter, and optionally the at least one particle shape parameter is determined.

15. Method according to claim 14, whereby, if the deviation is above a predetermined threshold, a warning is provided to the user and/or whereby a digital image and/or an outline image giving rise to diverging parameters is identified and/or indicated.

16. Method according to any of preceding claims, whereby the at least one particle size parameter comprises at least one statistical parameter selected from the group of average particle size, mean diameter, Dₓ-value with x = 1 - 100 and/or fineness modulus.

17. Method according to any of preceding claims, whereby the at least one particle size parameter extracted comprises a deviation from a predefined nominal value and/or nominal distribution.

18. Method according to any of preceding claims, whereby, for particle sizes below the minimum detectable particle size, the particle size distribution is extrapolated based on the extracted particle size distribution.

19. Method according to any of preceding claims, whereby the at least one particle shape parameter extracted comprises the roundness, sphericity, aspect ratio, roughness, solidity, flakiness index, shape index, percentage of crushed and broken surfaces, and/or angularity.

20. Method according to any of preceding claims, whereby the at least one particle shape parameter is extracted with regard to particles of a predetermined size only, especially to particles larger than a given threshold size, or whereby, for each of at least two or more predefined size fractions of the particles, at least one individual particle shape parameter is extracted, especially at least one individual mean value of the particle shape parameter is extracted.

21. Method according to any of preceding claims, comprising the step of assigning at least one attribute to the sample of solid particles.

22. Method according to any of preceding claims, whereby the method is at least partly, especially completely, performed on the mobile computer device.

23. Method according to any of preceding claims, whereby the image analysis in step c) and/or the making available in step d) is/are conducted on a separate computer device, e.g. on a server.

24. Method according to any of preceding claims, whereby the image, preferably together with the at least one attribute, and optionally the outline image, is stored on an external computer device, e.g. a server, in particular for sharing, recalling and/or further evaluation.

## Patentansprüche

1. Computerimplementiertes Verfahren zum Charakterisieren von festen Partikeln, vor allem von Sandpartikeln, umfassend die Schritte:
a) Bereitstellen einer Probe von zu analysierenden festen Partikeln in einer vordefinierten Probefläche;
b) Aufnehmen mindestens eines Digitalbildes der Probe von festen Partikeln mit einer Kamera einer mobilen Computervorrichtung;
c) Ausführen einer Abbildungspartikelanalyse des mindestens einen Digitalbildes zum Extrahieren von mindestens einem Partikelgrößenparameter und optional mindestens einem Partikelformparameter der Population von in dem mindestens einen Digitalbild identifizierten Partikeln;
d) Zurverfügungstellung des mindestens einen Partikelgrößenparameters und optional des mindestens einen Partikelformparameters über eine Benutzerschnittstelle, über eine Maschinenschnittstelle und/oder über ein Datenspeichermedium,
**dadurch gekennzeichnet, dass** in Schritt a) die Probefläche eine zweidimensionale Probefläche ist, die horizontal ausgerichtet ist und wobei die festen Partikel in einer einzelnen Schicht angeordnet sind und/oder derart, dass im Wesentlichen keine Überlappung von Partikeln in der Probefläche vorliegt.

2. Verfahren nach Anspruch 1, wobei die mobile Computervorrichtung ein Human Interface Device umfasst, insbesondere umfassend eine Eingabevorrichtung und eine Anzeige und vorzugsweise eine Drahtloskommunikationsvorrichtung.

3. Verfahren nach einem der vorhergehenden Ansprüche, wobei die mobile Computervorrichtung ausgewählt ist aus einem Mobiltelefon, einem mobilen Computer oder einem tragbaren Computer und einem Head-Mounted Display mit Kamera.

4. Verfahren nach einem der vorhergehenden Ansprüche, wobei die Kamera eine Kamera zum Aufnehmen von Bildern im sichtbaren Spektrum ist, vor allem von Farbbildern.

5. Verfahren nach einem der vorhergehenden Ansprüche, wobei die Kamera eine Auflösung von mindestens 2 Megapixel hat, insbesondere mindestens 5 Megapixel, vorzugsweise mindestens 8 Megapixel, insbesondere mindestens 12 Megapixel, stark bevorzugt mindestens 20 Megapixel, noch stärker bevorzugt mindestens 50 Megapixel oder mindestens über 100 Megapixel.

6. Verfahren nach einem der vorhergehenden Ansprüche, wobei die Probefläche eine Referenzskala umfasst und/oder eine bekannte Größe aufweist.

7. Verfahren nach einem der vorhergehenden Ansprüche, wobei die vordefinierte Probefläche ein dünnes Plattenmaterial ist, insbesondere eines mit vordefinierter Größe.

8. Verfahren nach einem der vorhergehenden Ansprüche, wobei die Partikel der Probe ausgewählt sind aus Sand, Aggregaten, Fasern und Glaskugeln.

9. Verfahren nach einem der vorhergehenden Ansprüche, wobei beim Aufnehmen des Bildes die Kamera so ausgerichtet wird, dass ein Anteil der
Probefläche in dem Bild maximiert wird, insbesondere durch Bereitstellen von Ausrichtungsanweisungen für den Benutzer und/oder durch automatisches Einstellen mindestens einer Einstellung der Kamera, z. B. der Brennweite der Kamera.

10. Verfahren nach einem der vorhergehenden Ansprüche, wobei eine Mindestgröße von nachweisbaren Partikeln berechnet wird durch das Einbeziehen der Auflösung der Kamera, des Längenanteils der Probefläche an der Gesamtfläche des Bildes und der realen Länge der Probefläche.

11. Verfahren nach einem der vorhergehenden Ansprüche, wobei, wenn eine kleinste nachweisbare Partikelgröße unter einem vorbestimmten Schwellenwert liegt, eine Warnung an den Benutzer ausgegeben wird, Ausrichtungsanweisungen an den Benutzer ausgegeben werden und/oder eine Einstellung der Kamera, z. B. die Brennweite, automatisch eingestellt wird.

12. Verfahren nach einem der vorhergehenden Ansprüche, wobei der mindestens eine extrahierte Partikelgrößenparameter die Partikelgrößenverteilung der Population von in dem mindestens einen Digitalbild identifizierten Partikeln umfasst.

13. Verfahren nach einem der vorhergehenden Ansprüche, wobei für jedes des mindestens einen Digitablildes ein Umrissbild erzeugt wird und vorzugsweise das Umrissbild in Schritt d) über eine Benutzerschnittstelle, über eine Maschinenschnittstelle und/oder auf einem Datenspeichermedium verfügbar gemacht wird.

14. Verfahren nach einem der vorhergehenden Ansprüche, wobei in Schritt b) mindestens zwei, vorzugsweise drei, insbesondere mindestens fünf oder mindestens zehn Digitalbilder aufgenommen werden und für jedes Bild in Schritt c) eine Abbildungspartikelanalyse durchgeführt wird und wobei durch Einbeziehen jedes des mindestens einen Partikelgrößenparameters und optional des mindestens einen Partikelformparameters, individuell extrahiert aus den mindestens zwei Bildern, eine Abweichung, insbesondere die Standardabweichung des mindestens einen Partikelgrößenparameters und optional des mindestens einen Partikelformparameters bestimmt wird.

15. Verfahren nach Anspruch 14, wobei, wenn die Abweichung über einem vorbestimmten Schwellenwert liegt, eine Warnung an den Benutzer ausgegeben wird und/oder wobei ein Digitalbild und/oder ein Umrissbild, das zu abweichenden Parametern führt, identifiziert und/oder angezeigt wird.

16. Verfahren nach einem der vorhergehenden Ansprüche, wobei der mindestens eine Partikelgrößenparameter mindestens einen statistischen Parameter umfasst, ausgewählt aus der Gruppe bestehend aus durchschnittlicher Partikelgröße, durchschnittlichem Durchmesser, Dₓ-Wert mit x = 1 - 100 und/oder Feinheitsmodul.

17. Verfahren nach einem der vorhergehenden Ansprüche, wobei der mindestens eine extrahierte Partikelgrößenparameter eine Abweichung von einem vordefinierten Nennwert und/oder von einer Nominalverteilung umfasst.

18. Verfahren nach einem der vorhergehenden Ansprüche, wobei für Partikelgrößen unterhalb der kleinsten nachweisbaren Partikelgröße die Partikelgrößenverteilung basierend auf der extrahierten Partikelgrößenverteilung extrapoliert wird.

19. Verfahren nach einem der vorhergehenden Ansprüche, wobei der mindestens eine extrahierte Partikelformparameter Rundheit, Kugelform, Seitenverhältnis, Rauheit, Festigkeit, Flockenindex, Formindex, Prozentsatz zerdrückter und gebrochener Oberflächen und/oder Kantigkeit umfasst.

20. Verfahren nach einem der vorhergehenden Ansprüche, wobei der mindestens eine Partikelformparameter nur in Bezug auf Partikel mit einer vorbestimmten Größe extrahiert wird, insbesondere auf Partikel größer als eine vorgegebene Schwellengröße oder wobei für jede von mindestens zwei oder mehr vordefinierten Größenfraktionen der Partikel mindestens ein individueller Partikelformparameter extrahiert wird, insbesondere wobei mindestens ein individueller Mittelwert des Partikelformparameters extrahiert wird.

21. Verfahren nach einem der vorhergehenden Ansprüche, umfassend den Schritt des Zuordnens mindestens eines Attributs zu einer Probe von festen Partikeln.

22. Verfahren nach einem der vorhergehenden Ansprüche, wobei das Verfahren mindestens teilweise, insbesondere vollständig auf der mobilen Computervorrichtung ausgeführt wird.

23. Verfahren nach einem der vorhergehenden Ansprüche, wobei die Bildanalyse in Schritt c) und/oder die Zurverfügungstellung in Schritt d) auf einer separaten Computervorrichtung ausgeführt wird/werden, z. B. auf einem Server.

24. Verfahren nach einem der vorhergehenden Ansprüche, wobei das Bild, vorzugsweise gemeinsam mit dem mindestens einen Attribut, und optional dem Umrissbild auf einer externen Computervorrichtung, z. B. auf einem Server gespeichert werden, insbesondere zum Teilen, zum Aufrufen und/oder zur weiteren Auswertung.

## Revendications

1. Procédé mis en œuvre par ordinateur pour caractériser des particules solides, en particulier des particules de sable, comprenant les étapes suivantes :
a) mise en place d'un échantillon de particules solides à analyser dans une zone d'échantillon prédéfinie ;
b) prise d'au moins une image numérique de l'échantillon de particules solides avec une caméra d'un dispositif informatique mobile ;
c) réalisation d'une analyse de particules par imagerie sur l'au moins une image numérique pour extraire au moins un paramètre de taille de particules, et éventuellement au moins un paramètre de forme de particules, de la population de particules identifiées dans l'au moins une image numérique ;
d) mise à disposition de l'au moins un paramètre de taille de particules, et éventuellement de l'au moins un paramètre de forme de particules, par le biais d'une interface utilisateur, par le biais d'une interface machine et/ou sur un support de stockage de données,
**caractérisé en ce qu'**à l'étape a), la zone d'échantillon est une zone d'échantillon bidimensionnelle qui est alignée horizontalement et dans lequel les particules solides sont disposées en une seule couche et/ou de telle sorte qu'il n'y a pratiquement pas de chevauchement de particules dans la zone d'échantillon.

2. Procédé selon la revendication 1, dans lequel le dispositif informatique mobile comprend un dispositif d'interface humaine, comprenant en particulier un dispositif d'entrée et un écran, et de préférence une interface de communication sans fil.

3. Procédé selon l'une quelconque des revendications précédentes, dans lequel le dispositif informatique mobile est choisi parmi un téléphone mobile, un ordinateur transportable et un ordinateur portable, et/ou un visiocasque avec caméra.

4. Procédé selon l'une quelconque des revendications précédentes, dans lequel la caméra est une caméra destinée à prendre des images dans le spectre visible, en particulier des images en couleur.

5. Procédé selon l'une quelconque des revendications précédentes, dans lequel la caméra possède une résolution d'au moins 2 mégapixels, en particulier au moins 5 mégapixels, de préférence au moins 8 mégapixels, particulièrement au moins 12 mégapixels, mieux au moins 20 mégapixels, mieux encore au moins 50 mégapixels ou au moins plus de 100 mégapixels.

6. Procédé selon l'une quelconque des revendications précédentes, dans lequel la zone d'échantillon comprend une échelle de référence et/ou a une taille connue.

7. Procédé selon l'une quelconque des revendications précédentes, dans lequel la zone d'échantillon prédéfinie est un matériau en feuille mince, en particulier d'une taille prédéfinie.

8. Procédé selon l'une quelconque des revendications précédentes, dans lequel les particules de l'échantillon sont choisies parmi le sable, les agrégats, les fibres et/ou les sphères de verre.

9. Procédé selon l'une quelconque des revendications précédentes dans lequel, lors de la prise de l'image, la caméra est alignée de telle sorte qu'une part de la zone d'échantillon dans l'image est maximisée, en particulier par la fourniture d'instructions d'alignement à l'utilisateur et/ou par ajustement automatique d'au moins un réglage de la caméra, par ex. de la longueur focale de la caméra.

10. Procédé selon l'une quelconque des revendications précédentes, dans lequel une taille de particules détectable minimale est calculée en tenant compte de la résolution de la caméra, de la part de la longueur de la zone d'échantillon dans la superficie totale de l'image, et de la longueur réelle de la zone d'échantillon.

11. Procédé selon l'une quelconque des revendications précédentes dans lequel, si une taille de particules détectable minimale est inférieure à un seuil prédéterminé, un avertissement est fourni à l'utilisateur, des instructions d'alignement sont fournies à l'utilisateur et/ou un réglage de la caméra, par ex. la distance focale de la caméra, est ajusté automatiquement.

12. Procédé selon l'une quelconque des revendications précédentes, dans lequel l'au moins un paramètre de taille de particules extrait comprend la distribution de taille de particules de la population de particules identifiées dans l'au moins une image numérique.

13. Procédé selon l'une quelconque des revendications précédentes dans lequel, pour l'image numérique ou chacune des images numériques, une image de contour est générée, et de préférence l'image de contour est mise à disposition à l'étape d) par le biais d'une interface utilisateur, par le biais d'une interface machine et/ou sur un support de stockage de données.

14. Procédé selon l'une quelconque des revendications précédentes dans lequel, à l'étape b), au moins deux, de préférence au moins trois, en particulier au moins cinq ou au moins dix images numériques sont prises, et pour chaque image, une analyse de particules par imagerie est réalisée à l'étape c), et en tenant compte du paramètre ou de chacun des paramètres de taille de particules, et éventuellement de l'au moins un paramètre de forme de particules, extraits individuellement des au moins deux images, un écart, en particulier l'écart type, de l'au moins un paramètre de taille de particules, et éventuellement de l'au moins un paramètre de forme de particules, est déterminé.

15. Procédé selon la revendication 14 dans lequel, si l'écart est supérieur à un seuil prédéterminé, un avertissement est fourni à l'utilisateur et/ou dans lequel une image numérique et/ou une image de contour donnant lieu à des paramètres divergents sont identifiées et/ou indiquées.

16. Procédé selon l'une quelconque des revendications précédentes, dans lequel l'au moins un paramètre de taille de particules comprend au moins un paramètre statistique choisi dans le groupe constitué par la taille moyenne de particules, le diamètre moyen, la valeur Dₓ avec x = 1 - 100 et/ou le module de finesse.

17. Procédé selon l'une quelconque des revendications précédentes, dans lequel l'au moins un paramètre de taille de particules extrait comprend un écart par rapport à une valeur nominale et/ou distribution nominale prédéfinie.

18. Procédé selon l'une quelconque des revendications précédentes dans lequel, pour les tailles de particules inférieures à la taille de particules détectable minimale, la distribution de taille de particules est extrapolée sur la base de la distribution de taille de particules extraite.

19. Procédé selon l'une quelconque des revendications précédentes, dans lequel l'au moins un paramètre de forme de particules extrait comprend la rondeur, la sphéricité, le rapport de forme, la rugosité, la solidité, l'indice de friabilité, l'indice de forme, le pourcentage de surfaces broyées ou cassées, et/ou l'angularité.

20. Procédé selon l'une quelconque des revendications précédentes, dans lequel l'au moins un paramètre de forme de particules est extrait uniquement pour les particules d'une taille prédéterminée, en particulier pour les particules plus grosses qu'une taille seuil donnée, ou dans lequel, pour chacune d'au moins deux fractions de taille prédéfinies des particules, au moins un paramètre de forme de particules individuel est extrait, en particulier au moins une valeur moyenne individuelle du paramètre de forme de particules est extraite.

21. Procédé selon l'une quelconque des revendications précédentes, comprenant l'étape d'attribution d'au moins un attribut à l'échantillon de particules solides.

22. Procédé selon l'une quelconque des revendications précédentes, le procédé étant au moins partiellement, en particulier entièrement, réalisé sur le dispositif informatique mobile.

23. Procédé selon l'une quelconque des revendications précédentes, dans lequel l'analyse d'image à l'étape c) et/ou la mise à disposition à l'étape d) est/sont conduite(s) sur un dispositif informatique séparé, par ex. sur un serveur.

24. Procédé selon l'une quelconque des revendications précédentes, dans lequel l'image, de préférence conjointement avec l'au moins un attribut, et éventuellement l'image de contour, est stockée sur un dispositif informatique externe, par ex. un serveur, en particulier pour un partage, un rappel et/ou une autre évaluation.
